# EUROPEAN PATENT APPLICATION

(11) **EP 1 746 418 A2**
(43) Date of publication of application: **24.01.2007**
(21) Application number: 06253768.3
(22) Date of filing: 19.07.2006
(51) Int. Cl.: G01N 33/38, G01N 29/44

(54) **An inspection device**

(30) Priority: 20.07.2005 GB 0514921
(71) Applicant: Andrews, David Richard, Cambridge CB4 5NX (GB)
(72) Inventor: Andrews, David Richard, Cambridge CB4 5NX (GB)

(57) **Abstract**

An inspection device uses sound waves or ultrasound waves to generate images of the interior of structures under test, with particular application to heterogeneous materials, such as concrete. Images of the interior of the structure under test are created by repeatedly combining signals from transmitters and receivers at a plurality of different locations to reduce the effect of random scattering from the grain particles in the heterogeneous material. The device includes means for efficiently managing the movement of transducers between different test locations so that tests can be done quickly and consequently at low-cost and with means to process echo-signals to create images.

## Description

The present invention relates to a device for inspecting the mechanical integrity and operational worthiness of structures, particularly structures made of heterogeneous materials for example: concrete structures, cast iron components and austenitic stainless steel components. Examples of large structures that can be inspected are: bridges, power stations and dams.

Large structures are expensive to build and they are generally designed to last between twenty years and one hundred years. Many structures of this kind have been built since 1950 and owners and operators of these structures have a strong financial incentive to prolong using them and to avoid demolishing them when they reach the end of their design-life. There is also a significant environmental cost associated with demolishing and rebuilding which it is desirable to avoid. Consequently, it is desirable to prolong using structures of this kind for as long as possible.

It is also desirable to use structures with reduced material quantities because this brings down the cost of construction, however, reduced material quantities can result in lower margins of safety.

When using a structure beyond its design life or with lower safety margins it becomes particularly important to inspect and to monitor its mechanical integrity and to review its operational worthiness more closely in order to maintain safe operating conditions. It is also financially advantageous to direct maintenance and repair to locations with the most important or significant deterioration.

Clearly the quality of inspection is important when reviewing the operational worthiness of ageing structures and lightweight structures. Current inspection procedures typically involve a visual examination by an experienced structural engineer. Many forms of deterioration of structures are invisible from the surface of a structure, for example the corrosion of steel tendons in post-tensioned, reinforced concrete structures. Many experts consider visual inspection to be inadequate.

It would be desirable if an inspection device could reveal the following information in concrete structures: positions of steel forms, regions of poor quality concrete, regions of corroded steel forms, regions of corroded steel inside tendon ducts. It would be desirable if an inspection device could reveal all those things whilst working from a single testing surface. It would also be desirable if the inspection device could measure the thickness of concrete beams. It would be particularly desirable if the inspection device could display this information quickly and in the form of an image that could be viewed at the time of testing so that the operator of the inspection device could adapt the test conditions to get as much information as is needed or as is possible to make clear the structural integrity of the internal structure.

### STATE OF THE ART

Structures made from steel reinforced concrete get most of their strength from the steel forms therein and the concrete exists mainly to protect the steel from corroding due to the environment. Since the steel forms are embedded in concrete the steel cannot be inspected visually.

It is known to inspect or monitor concrete structures by a number of methods: thermography, gamma ray, X-radiography, vibration holography, neutron radiography, dye penetration, magnetic induction, electric potential mapping, radar, acoustic emission, ultrasonic time of flight, ultrasonic resonance spectroscopy, ultrasonic pulse-echo, ultrasonic guided-wave, and electromagnetic guided-wave testing. Many of these methods have disadvantages; for example, radar, or microwaves, cannot penetrate the metal duct surrounding post-tensioned tendons. X-rays can penetrate the tendon duct and detailed images of steel forms in concrete can be made but exposure times for creating an image are typically several hours and it may not be possible to keep the structure fully operational during exposure because of the radiation hazard inherent with X-rays.

Sound waves and ultrasonic waves, referred to herein collectively as sound waves, are inherently sensitive methods for probing virtually all structural materials, including concrete. Because they are mechanical waves, sound waves are intrinsically sensitive to the mechanical composition of any material; by way of contrast, electromagnetic waves are sensitive to changes in the electrical properties of materials and may, therefore, only be indirectly sensitive to mechanical properties. Sound waves are known for testing steel and many other metals but these are generally, substantially homogenous materials. Concrete is a heterogeneous material, it is not a homogeneous material so conventional sound wave methods, using substantially plane waves and phase-sensitive, coherent transducers, do not work well on concrete.

It is known to test a range of materials using sound or ultrasound using one or two and sometimes more electro-mechanical conversion devices referred to herein as transducers, by which means sound waves or ultrasound is first caused to be emitted into the material under test using a transmitter transducer and echoes from the material are collected by a receiver transducer and converted into a convenient electrical signal which is displayed to an operator. It is known to test heterogeneous materials, materials with a significant internal grain structure, such as concrete with large aggregate constituents, using ultrasonic pulse-echo equipment with two ultrasonic transducers frequently made with relatively large apertures used in an A-scan, or amplitude against time representation of the received signal; in this case the transducers are made large in an attempt to collect more ultrasonic energy. However, the quality and quantity of information from these tests is generally poor and they are of limited usefulness. The roughness of most concrete surfaces results in relatively few points of contact across the apertures of the transducers, which itself results in a significant reduction of energy transmitted and received. It is possible to use the impact of a ball onto the concrete surface as an alternative transmitter and to use a small, point-contact receiver but the usefulness of the test remains substantially less than inspection engineers would like.

It is also known to test concrete using a planar array of approximately twenty transducers with spring-loaded, point-contact, shear-wave transducers wired permanently in a fixed phase relationship, with one or more transducers used as transmitters and of the remainder one or more used as receivers. All of these transducers share a common feature that they combine the signals from ultrasonic waves received immediately upon arrival over the relatively large area of the aperture. This works well when a wave with plane wave-fronts is received parallel to the plane of the transducer. However, plane waves are seldom received in heterogeneous materials even if plane waves are launched by the transmitter or transmitters. The reason plane waves cannot exist in heterogeneous materials is because the randomly distributed grains, which are the important characteristic of heterogeneous materials, cause random scattering of the sound or ultrasound waves passing through them and this results in partial disruption of the coherence of the ultrasonic wave-fronts. What arrives at the transducer is not a plane wave-front but a partially randomized pulse of spherical wavelets with partly random phase differences. This scattering is only significant if the wavelength of the sound wave or ultrasound wave is commensurate with or smaller than the largest grain size. All materials become heterogeneous if the wavelength is sufficiently small and, consequently, the frequency is sufficiently high. Many materials can be tested adequately away from this condition and then the materials appear homogeneous and random scattering is not a significant effect.

It is common in the ultrasonic inspection industry to use a phase-sensitive receiver of either a single large aperture receiver (larger than the wavelength) or an array of point receivers with fixed phase-sensitive combination of signals, frequently simple addition of signals. However, when the material is behaving heterogeneously, randomly ordered wavelets arrive at phase-sensitive receivers and try to cause randomly localized electrical charges of random polarity and value which combine destructively within the receiver, resulting in a signal level much smaller than would be achieved with plane waves parallel to the plane of the receiver. In an extreme case, it is possible for the output signal of a phase-sensitive receiver to have zero amplitude, despite the arrival on it of a powerful but disrupted packet of sound waves. Moreover, the larger the aperture or the more transducers in a phase-sensitive receiver the smaller the signal will probably be because there is a greater probability of large phase differences, resulting in almost total destructive interference. The inspection device described herein avoids this problem by using a plurality of transmitters and receivers, each with an aperture no greater than the wavelength of ultrasound used in the test, ensuring minimal phase cancellation at each receiver, and combining the signals in a phase insensitive way, only allowing constructive interference, thereby maximizing the signal collected and avoiding loss of information.

Examples of materials that are heterogeneous under the conditions for which inspection is desirable using sound or ultrasound waves are: concrete, cast iron and austenitic stainless steel.

It is known that many ultrasonic inspection devices apply a sharp high voltage spike to a transmitter thereby causing the transmitter to launch an ultrasonic wave of short duration; nearly all commercial ultrasonic inspection systems try to keep the ultrasonic wave pulse as short as possible. The transmitted wave pulse is generally reflected within the sample under test with an echo returning to a receiver. When this echo rises above a fixed threshold it is judged to be significant for the purpose of interpretation. Experience shows that fixed threshold levels do not give good results with heterogeneous materials, in the presence of random scattering by grains, because those grains close to the surface and immediately under a transmitter invariably contribute a stronger echo than more distant reflectors so that the operator has a problem to detect echoes of interest. Spike excitation has a number of other disadvantages: the energy efficiency of creating sound or ultrasound waves is poor because much of the energy in the spike is outside the frequency range of sound or ultrasound waves that can be generated by a transducer; consequently, unused energy in the spike can generate radio frequency interference; relatively low signal-to-noise ratio in any receiver signal, since it is the energy in the ultrasonic wave that contributes to the signal-to-noise ratio. Other forms of excitation are possible that do not have these disadvantages but which require more sophisticated signal processing of received signals, for example pattern recognition of chirps.

A sound wave in a solid can be of two main types: compression and shear. A compression wave travels with particle motion parallel to the direction in which the wave is moving; a shear wave travels with particle motion perpendicular to the direction in which the wave is moving. There is only one direction of particle motion possible for a compression wave, it is sometimes known as a scalar wave for this reason but for a shear wave the direction of particle motion can be at any angle to the direction of the wave so it is common to refer shear waves as vector waves. A shear or vector wave has a direction of polarization referred to the direction of any two, convenient, perpendicular vectors, each of which is perpendicular to the direction of the wave. It is also possible to have waves associated with surfaces and hybrid waves which are a collection of more than one fundamental type of wave. By way of contrast, liquids only support compression, not shear, so in a liquid there are only compression waves.

It is known that different types of waves travel at different speeds, particularly compression and shear waves and it is found that shear waves generally have a wave speed of roughly 60% of the speed of compression waves. The existence of these different wave types presents a difficulty for the inspection of solid materials with sound waves because, for example, a crack in a metal can reflect a compression wave and generate a shear wave at its tip, thereby generating two waves from approximately the same place that will travel back to a receiver. If the receiver transducer were sensitive to both compression and shear then there would be two peaks in the electrical signal from the receiver; this may be acceptable when testing a single flaw in an otherwise pristine sample, with no other echoes, but the presence of even a few other echoes quickly makes the situation confusing for the operator: which echoes come from the same reflector or flaw? In most commercial inspection systems the situation is simplified by using a single transducer to both transmit and receive that is more sensitive to one of the wave types than to the other. Information from the other wave type is ignored but this is potentially valuable information. In the inspection device described here it is possible to transmit either compression or two orthogonal polarizations of shear waves and to receive using compression or the two orthogonal polarizations; the inspection device does not discard any information and permits information from different wave types to be viewed by the operator.

It is known in the medical application of ultrasound inspection to create images based upon harmonics of the transmitted ultrasonic wave and this sometimes gives improved contrast to changes in tissue. Harmonics are created when a sonic or ultrasonic waves passes through a material that is non-linear or through an interface that is non-linear, such as a crack. It is known in the medical application of ultrasound inspection that certain objects such as metallic objects, with substantially different acoustic properties to the surrounding tissue, can resonate. Also submarines resonate when detected by sonar and the resonances can be used to identify the submarine to some degree.

It is known to image the interior of objects by focusing sonic or ultrasonic waves. An array of transducers in one or two dimensions, can be used to form an image of the interior of a body using all of the transducers in the array, or at least a majority or plurality of them, for simultaneously transmitting and simultaneously receiving; by using a range of appropriate delays between electrical excitations to transmitters it is possible to cause the waves transmitted into the object to interfere to form different wave patterns therein, for example: plane waves, plane waves angled relative to the array, focused waves that focus at an arbitrary position within some range of the array. It is likewise possible to collect received signals and cause them to combine, using appropriate delays, to form a signal that appears to have emerged from a focal point in the body. So there are two distinct possibilities: transmit focusing and receive signal focusing; the former is a physical sonic or ultrasonic wave effect, the latter is a virtual or synthetic effect that can only occur within an algorithm. The disadvantage of arrays of many elements is that they require a plurality of complex, electronic channels both for transmitting and receiving, sometimes as many as 128 or 256 channels, which result in bulky, heavy and expensive equipment that cannot easily be held by hand. It is desirable to have so many channels working simultaneously so that a detailed image can be created 30 times per second and the human brain can see a persistent image; the imaging system can then be used to advantage on the human body to image tissue moving therein. It is not important to have an inspection device for rigid structures that can produce images at 30 times per second when the structure is substantially unchanging over times as short as one second. The present invention seeks to apply some benefits of signals obtained at many positions, particularly benefits that relate to imaging in heterogeneous materials, but in a device that is as small, as lightweight as possible and which results in a slower imaging speed; it is possible to have an imaging system with many fewer electronic channels, building-up an image more slowly and not using transit focusing methods.

### STATEMENT OF INVENTION

Accordingly, in a device for inspecting the mechanical integrity and operational worthiness of structures, including structures made of heterogeneous materials, using sound waves or ultrasound waves and their echoes from components within the structure, there is provided: (a) means for geometrically arranging one or more but few sonic or ultrasonic electro-mechanical conversion transducers, (b) means to select any of the transducers and means to cause any one or a few at a time of the selected transducers to generate sound waves and/or ultrasound waves and means to select any of the transducers and means to collect electrical signals received from any one or a few at a time of the selected transducers caused by the impingement of sound waves and/or ultrasound waves echoing from the structure under inspection; (c) the aforementioned transducers with some or possibly all able to generate and/or receive substantially compression sound waves or ultrasound waves, or with possibly some or possibly all of the aforementioned transducers able to generate and/or receive substantially shear sound waves or ultrasound waves; (d) with the direction or plane of shear polarization of the aforementioned shear-transducers all being known and constrained either parallel or of a known pattern of polarization between each individual transducer; (e) each aforementioned transducer with an aperture that is preferably not larger and possibly much smaller in actuating dimension than a representative wavelength of sound wave and/or ultrasound wave used for inspection; (f) all the aforementioned transducers with average actuating surface level, when in use, being possibly co-planar or possibly profiled to match the profile of the surface of the structure to be inspected, and possibly with means to adapt the profile to the profile of the surface of the structure; (g) the aforementioned transducers provided with means to register the position of each transducer on the surface of the structure when a test is performed so that this information can be used along with the received echoes from the structure to form images of the interior of the structure; (h) the aforementioned transducers provided with means to allow them to move or slide substantially perpendicular to the surface under test and to be engaged with and to disengage from the testing surface of the structure under inspection; (i) means to couple or transfer sound wave or ultrasound wave energy between each aforementioned transducer and the surface of the structure under inspection, when engaged thereupon; (j) means for creating and applying excitation patterns to a transducer selected to transmit; (k) means for collecting and storing representations of signals received by selected transducers; (1) means for processing the signals from receivers to create an image to represent the interior of the structure under test based upon echoes from sound waves or ultrasound waves emitted into the structure by the inspection device; (m) means to display information about the interior of the structure under test for an operator to view; (n) means to allow an operator to control the conditions of testing; (o) means to provide power to the inspection device; (p) means to allow the inspection device to be used quickly and easily to inspect a structure.

### CONSISTORY CLAUSES

An inspection of a structure using the inspection device described herein could take several hours or days or possibly more to create an image if the device were heavy or slow to process information because the creation of an image can make use of a large number of echo signals which give rise to a large number of mathematical operations. Consequently, it is desirable but not essential that the inspection device should be portable, lightweight and capable of performing tests quickly and continuing to work for several hours.

To increase the speed of testing it is desirable but not essential to have an inspection device with a group or groups of transducers mechanically held together in one or possibly more housings, to make transducer assemblies, such that a group consists of at least two or more transducers in a line, with a space between each of approximately but not necessarily one transducer width or a space between each commensurate with the wavelength of sound wave or ultrasound wave used in testing.

To increase the speed of testing further it is desirable but not essential to have an inspection device with two or preferably three groups of transducers mechanically held together in a housing, to make a transducer assembly, such that each group consists of at least two or more transducers in a line, with a space between each, and the two or three lines parallel and kept close together. One group of transducers would preferably but not essentially be substantially sensitive to compression waves, a second group of transducers would be preferably sensitive to shear waves each with their plane of polarization parallel to the line of the transducers, a third group of transducers would be preferably sensitive to shear waves each with their plane of polarization perpendicular to the line of the transducers. In this preferred embodiment the inspection device can be operated in nine different inspection modes: transmitting compression waves and receiving shear parallel waves, transmitting compression waves and receiving shear perpendicular waves, transmitting compression waves and receiving compression waves, transmitting shear parallel waves and receiving compression waves, transmitting shear parallel waves and receiving shear perpendicular waves, transmitting shear parallel waves and receiving shear parallel waves, transmitting shear perpendicular waves and receiving compression waves, transmitting shear perpendicular waves and receiving shear parallel waves and transmitting shear perpendicular waves and receiving shear perpendicular waves.

It is possible to use some mechanical lifting device or arm to move some or all of, possibly but not necessarily, heavier embodiments of the inspection device, such as those with many transducers in each transducer assembly. An arm in the form of a pantograph can also be used to register the position and orientation of the transducer assembly relative to the surface under test.

The transducer assembly in a portable inspection device should preferably but not necessarily be capable of being held by an operator by hand so it is preferable that it should be lightweight. Consequently, it is preferable but not essential that some or most of any electrical circuits and any battery or batteries are not held in or on the transducer assembly but are instead held in a separate unit, possibly but not necessarily in a unit carried on the back of the operator.

Since the weight of the transducer assembly depends upon the number of transducers therein and the individual weight of a transducer, it is preferable but not essential to have relatively few transducers and they should be lightweight. A preferred embodiment has an array of approximately four to ten transducers in a line (a linear array) but other numbers are possible. A linear array is preferred for use on concrete structures not only because it has relatively few transducers but also because many steel forms used in concrete structures also have a linear shape and, consequently, a linear array can be placed over these linear forms and adjusted until it can be deduced to be parallel to or perpendicular to the line of the steel form, thereby discovering the orientation of the steel form from the surface.

The surface of the structure to be tested can have various profiles: substantially flat or planar and cylindrical are both common profiles but other profiles are also possible. It is desirable that an inspection device can work on a variety of surface profiles. A single transducer can be used on almost any profile but this is slow in use because the transducer has to be moved many times to build up a useful image of the interior of the structure; a planar array, with significant extent in two orthogonal directions, is suitable for testing substantially flat surfaces but it cannot be used on other profiles. A linear array can be used on flat surfaces and also on cylindrical profiles, provided the array is kept substantially parallel to the axis of the cylinder; a linear array can also be used on various other profiles if its length is small compared to the local radius of curvature and if its transducers can move perpendicular to the surface to some degree, preferably but not necessarily by approximately 10% of the length of the linear array. Some perpendicular motion is preferable in any case if the structure to be tested has a rough surface, like concrete.

Signals received from transducers at two or more positions can be usefully combined in an image to reduce substantially the otherwise obfuscating effect of random scattering of sound waves or ultrasound waves, this is a form of what is sometimes called spatial averaging. A preferred but not essential method for applying spatial averaging and creating an image has a focal plane chosen by an operator, divided into a plurality of focal points, possibly again chosen by the operator, and for each focal point in the focal plane as many stored echo signals as possible or desirable are processed by adding appropriate parts of them together with suitable time-delays chosen to compensate for the geometrical distances travelled to create an intensity at each focal point. By repeating the process for every focal point in the focal plane an image is developed over the entire focal plane which is relatively sharp. By setting a focal plane inside the structure and at different depths or orientations an image of the interior of the structure can be viewed by the operator.

It is known for heterogeneous materials that the degree of random scattering of sound waves or ultrasound waves from grains increases as the wavelength is made as small as the grain size; it is preferable for an inspection device to use sound waves or ultrasound waves with wavelengths greater than the grain size. A preferred embodiment of a transducer array to work on heterogeneous materials would have a size of at least one wavelength and preferably several wavelengths because echo-signals collected across an array of this size can be expected to be uncorrelated with respect to the grains through which the echoes have travelled; the echo-signals can then usefully be combined to average-out, or spatially average, the randomizing influence of the grains on sound or ultrasound waves and thereby reveal echoes from more distant features of interest. Concrete is commonly made with a range of aggregate sizes, or grain sizes, the largest being about 30 millimetre so that it is preferable to use sound waves or ultrasound waves with wavelengths longer than 30 millimetre. A preferred linear array transducer assembly for use on concrete is therefore several times longer than 30 millimetre. A preferred embodiment of an array for high strength concrete is more than 100 millimetre long and is preferably but not essentially in the range 100 millimetre to 500 millimetre long.

Another way to achieve spatial averaging is to move the transducer assembly to a different location and re-test and a preferred embodiment has means for moving the transducer assembly over the surface while simultaneously registering the degree, direction and orientation of each movement. Consequently, one possible embodiment of an inspection device, has at least one and preferably two or more wheels or rollers or track mechanisms or similar means of movement at either end of a transducer assembly. By bringing the wheels or similar means of movement into contact with the surface of the structure under test and moving the transducer assembly from test position to test position, with the wheels or similar means of movement always in slip-free contact with the surface, and with means for the degree of revolving or movement of the wheels or similar means of movement to be measured, it is possible to determine the relative displacement and any rotation of the transducer assembly between tests.

It is also possible to measure position and orientation of the transducer assembly on the surface under test using possibly one but preferably two or more sonic or ultrasonic transducers working in air and mounted on the transducer array and with two or more and preferably four sonic or ultrasonic transducers working in air fixed temporarily at two or more or preferably four known boundary positions on the surface of the structure, and suitably separated by distances to allow the transducer assembly to be used at as many positions as desired for one location of interest between the transducers fixed at the boundary. In the case of inspection of concrete the fixed transducers would typically but not necessarily be fixed about 2 m apart. The transducers have means to pass signals between them in air and by cable and electronic circuits and signal processing to determine the position of the transducer assembly using the time taken for sound waves or ultrasound waves to travel in air between the several transducers. Another way to measure position and orientation is to use radio waves or electromagnetic waves instead of ultrasound in air. It is important that the test locations of the array are found to an accuracy of better than about a quarter of the wavelength of the ultrasound used for inspecting the structure, which, for example, requires a precision of better than +/-10 millimetre and preferably +/-1 millimetre when testing concrete. The advantage of using ultrasound in air or radio waves or electromagnetic waves to determine the location of a transducer assembly is that the assembly can be removed from the test surface and returned with no loss of registration of position. It is preferable to use sound waves or ultrasound waves in air instead of radio waves or electromagnetic waves for measuring the position and orientation of the transducer assembly because the speed of sound in air is roughly 10⁶ times slower than the speed of electromagnetic waves and consequently the time for sound or ultrasound waves to travel between transducers in air is much longer and the electronic circuits required to measure the time delays are simpler, cheaper and generally more accurate.

The method of coupling or transferring ultrasonic energy between each transducer and the surface under test is important because it affects the quality and strength of the ultrasonic signals received and therefore, ultimately, the quality of the image provided by the inspection device for assessing the structure. The coupling method should be quick and easy to use, to keep testing times as short as possible, and to keep the cost of testing as low as possible. Point contact transducers can be used under favourable conditions, for example: a smooth test surface with little or no surface dust or friable material; but point contact transducers are intrinsically inefficient, in terms of the amount of energy that can be transferred between the transducer and the testing surface, so to increase sensitivity transducers are frequently made with little or no mechanical damping and this is well-known to result in transducers having a narrow working frequency range or bandwidth. A narrow bandwidth is a disadvantage because it is also well-known that narrow bandwidth results in a poor axial resolution for the transducer, which ultimately results in poor quality information about the structure. It is desirable to use transducers and signals with as wide a bandwidth as possible and therefore it is preferable to use transducers that transfer or couple ultrasonic energy over a larger area, which results in increased efficiency.

The largest preferred aperture size is approximately one wavelength of the highest frequency of sound or ultrasound used in a test; this preference results because it is desirable to minimise phase-sensitive interference effects occurring intrinsically over a receiver. By way of an example of how to select a suitable aperture size, using concrete as an example material but extending the principle to all heterogeneous materials, it has been found that test frequencies up to about 200 kHz give good results and assuming the wave speed is 4,000 metres per second in concrete a receiver aperture size of 20 millimetre or smaller is preferred. A preferred embodiment of an inspection device for use on concrete has a diameter of the transducer not greater than 50 millimetre and preferably about 20 millimetre.

The surface roughness of the test surface, for example concrete, can also result in low efficiency of coupling because sound or ultrasound waves can only travel through asperities or point contacts with a small area. Certain coupling agents can be used to improve the efficiency of coupling and for the specific example of concrete a preferred coupling material is a fast-setting mortar. Solid coupling agents such as adhesives transmit compression and shear waves equally well. Another class of preferred coupling agent is thin deformable materials and a preferred deformable material to use on concrete is silicone rubber filled with mineral fillers to increase the acoustic impedance and thereby improve the efficiency of coupling.

A preferred transducer for testing structures with rough surfaces has a substantially flat actuating surface and is made of a material with an acoustic impedance matched to the material of which the structure or the surface layer of the structure is made, with a small dome protruding from the flat surface of a height a little more than the size of the surface relief. In use the protrusion can fit into a suitable valley in the surface relief and generally couple over a larger surface area of the dome than would otherwise occur with an asperity of the surface in contact with the otherwise flat surface of the transducer.

A preferred transducer specifically for testing concrete has a substantially flat surface, made of a material with an acoustic impedance matched to concrete that adheres to mortars, with a small dome of a few millimetres in height protruding from it. This particular design can be used: for point-contact coupling, with liquid-gel coupling materials, with filled silicone rubber coupling materials and with fast-setting mortar, so that the operator has a choice of coupling material to use to suit the prevailing test requirements and conditions of the surface to be tested. The height of the protrusion is chosen to be commensurate with the surface roughness of common concrete surfaces.

It is preferable but not essential to use transducers so that they are not used simultaneously as receivers and transmitters because it is electrically impossible to transmit and to receive simultaneously and this results in a loss of information close to the contact surface under test.

It is preferable but not essential to complete testing at a location, to process the resulting signals and display the results to the operator or inspection engineer within a few seconds because the operator may be holding the transducer array in contact with the test surface waiting to assess the results before moving the array to another test location; even with a lightweight array there is a limit to how long an operator can hold a transducer in this way. It is desirable that the operator or inspection engineer should be able to assess the information resulting from any given test and to decide if testing at another location is desirable, so that an interactive process of re-testing at nearby locations can be used to help overcome the obfuscating effects of random scattering of sound waves and ultrasound waves in heterogeneous materials, for example concrete. An interactive approach results in an improved quality of inspection.

In order to minimise the cost of making the inspection device it is desirable but not essential to transmit from one transducer and digitize signals from one receiver transducer at a time, switching between all available transducers to select a transmitter and receiver. This approach substantially reduces the replication of electronic circuits in the form of transmit and receive channels that would otherwise be needed and hence reduces the cost and weight.

It is preferable but not essential in an inspection device to drive transmitter transducers with bursts of electrical excitation in a controlled pattern. It is preferable but not essential to use a swept-frequency chirp. A chirp is a burst of sine waves lasting more than one cycle during which time the frequency of the sine wave is changed. For example when testing concrete a suitable chirp could last for between 30 microseconds and 300 microseconds during which time the frequency could sweep over a range of frequencies linearly in time anywhere in the range from 200 kHz to 10 kHz. For inspecting stainless steel higher frequencies and shorter chirp durations are preferably used. Chirps can be chosen to suit particular materials, by which is meant that the statistics of the size distribution of grains in the material will tend to control what is the highest frequency that can be propagated without too much scattering; the range of frequencies should otherwise be as great as possible. The lowest frequency, or more importantly, the corresponding period of the lowest frequency, will tend to control the duration for the chirp, since a chirp with a duration shorter than the period of the lowest frequency will fail to explore or pronounce the chirp.

It is desirable to match the frequency range of any pattern of excitation used to drive transmitters to the bandwidth of the transducers, because this will help to maximise the sonic or ultrasonic efficiency of the inspection device and consequently its sensitivity; it is also desirable to use a pattern that can be received without too much distortion after travelling a distance of interest to the operator through the structure under test, generally lower frequencies have greater range but higher frequencies give better axial resolution.

When an inspection device is using a pattern of excitation of transmitters it is desirable to process signals received from echoes in the structure under test to detect occurrences of any known pattern, which converts the relatively long pattern into a shorter and sharper event signal and consequently results in sharper images. One preferred method is sometimes called matched filtering; it is the optimum linear signal processing method and it can make use of a copy of the transmitted excitation pattern. It is preferable, however, to make use of part of the signal transmitted during a calibrating test on a homogeneous sample, having the same or similar acoustic properties to the structural material of interest, because the original electrical excitation pattern is modified both by the transmitter as it is converted into a sonic or ultrasonic wave pattern and by the receiver as the sonic or ultrasonic pattern are collected and converted back into an electrical signal so the original excitation signal may not be an accurate copy of the pattern to be recognised and an experimentally collected chirp is generally more accurate. The more accurately the matched filter is matched to the pattern found in echo-signals from the structure under test the sharper and more accurate will be the resulting image generated from the echoes.

Even if spike excitation of the transmitters in the inspection device is used it is still preferable to use matched filtering based upon a pattern recorded in a calibration experiment, as described earlier here. With spike excitation most if not all transmitter transducers cause substantial distortion of the spike excitation because a spike covers a wide range of frequencies but the transmitter can only transmit waves in its, generally, much narrower bandwidth. The reduced bandwidth distorts the spike substantially and a pulse of waves is generated, which can still be efficiently detected by matched filtering.

An inspection device processes received signals in several stages one of which is to convert bipolar signals derived from echoes of the structure under test into unipolar signals. It is desirable to convert signals from a bipolar form to a unipolar form to eliminate phase sensitive combination of signals such as subtraction and thereby reduce the obfuscating effects of random scattering in heterogeneous materials. A preferred method is to calculate the magnitude of the analytic function of the signal. Other possible methods include simple rectification or taking the mathematical magnitude of the signal, these latter two are preferably but not essentially followed by low-pass filtering or band-pass filtering to smooth the signal. It is desirable that unipolar conversion follows matched filtering.

A preferred but not essential stage of processing after unipolar-conversion is to detect peaks in the signal, which are generally caused by echoes from strong reflectors in the structure under test. It is common in industrial inspection to apply a constant level threshold and detect peaks that exceed the threshold, allowing them to pass and setting to zero values elsewhere. This method does not work well when testing heterogeneous materials, for example concrete, which causes significant random scattering of ultrasonic waves during a test. It is preferable to use a time-varying threshold to detect peaks of significance and a preferred time-varying threshold is based upon the statistics of the random scatterers in the material under test, for example concrete. It is preferable but not essential to use the mathematical Weibull or Rayleigh distributions to create time-varying thresholds for unipolar signals; furthermore by adjusting the parameters of the chosen time-varying threshold it is possible to provide an image sensitivity control.

A preferred but not essential stage of additional processing is to identify any reverberations or resonances of sound waves or ultrasound waves emanating from a location in the structure under test and to measure the frequency of those resonances and display the resonances to the operator. For example in concrete, the degree of corrosion of stressing tendons or the existence of voids in a tendon duct may be detected by the presence of a resonance associated with the echo from the tendon. When an internal component is made of a different material from the surrounding matrix material, for example a tendon in concrete, it is possible for sound energy from the inspection device to be absorbed by the component and for that wave energy to be substantially trapped within the component, resulting in reverberations in it, and for reverberating echoes to leak back into the matrix. The way that the sound energy resonates will depend upon the dimensions of the component, the speed of waves within it, the coupling between its surfaces and the matrix material surrounding it; these factors in turn will be dependent upon the structural integrity of the component. Resonances can generally be located as emerging from certain locations or from certain components in a structure by timing measurements, the instantaneous resonant frequency or frequencies can be measured and from this information it is possible to display resonance results as emerging from a point in an image, possibly using colours or other graphical means. Presentation of the results in this way helps to draw the attention of the operator to the source of resonating or helps the operator to infer the qualities of the structure at the source of resonating.

A preferred but not essential stage of additional processing is to identify any harmonics of the excitation pattern. Harmonics are generally associated either with internal interfaces that are poorly bound mechanically, such as cracks, or dissimilar materials or certain materials that cause significant non-linear propagation of sound waves or ultrasound waves. If a pattern of excitation is used to drive transmitters in the inspection device then it is possible to construct a matched filter based upon any harmonic or sub-harmonic of the pattern. In this way images can be constructed based entirely upon harmonic generation or harmonic echoes can be shown superimposed at specific points upon the non-harmonic image. Presentation of the results in these ways helps to draw the attention of the operator to the source of harmonics or helps the operator to infer the qualities of the structure at the source of harmonics.

The inspection device preferably but not necessarily, contains a source of electrical energy, such as a re-chargeable battery or batteries, which can conveniently reside with the majority of the electrical circuits comprising the signal processing unit inside an enclosure. The enclosure is, preferably but not necessarily, sufficiently lightweight to be carried by the operator, preferably but not essentially, on the operator's back for periods of hours so that the operator is free to walk about the structure during an inspection, with negligible time spent in gathering-up parts of the inspection device. This arrangement helps to reduce the time spent on each test and helps to keep down the cost of an inspection.

It is also preferable for the operator to be able to view an image created from a test on a display panel on the inspection device, preferably but not necessarily while the operator is still holding the transducer assembly in contact with the concrete surface. It is preferable but not essential that the display panel is lightweight and mounted on the transducer assembly or, possibly, held onto the operator's head but viewable by the operator and can be adjusted in angle to suit the operator whilst the inspection device is in use.

It is preferable but not essential for the inspection device to have means to measure the speed of sound or ultrasound of compression and/or shear waves in the structure under test. It is preferable to use the inspection device on a part of the structure with a known dimension, for the inspection device to have means on it to allow the entry of the value of the dimension for processing and to perform a calibration test with the inspection device, using it to measure the time for an echo to return from a wall of the structure with a known distance from the transducer assembly and to use timing measurements on the echo to calculate the speed of sound. Speed equals the distance travelled through the structure divided by half of the time for the wave to pass from the transducer assembly and back; since in a reflection test the sound wave travels out and re-traces its path taking twice the time. It is also preferable to be able to connect a secondary or roving transmitter or transmitters of sound or ultrasound waves to the inspection device and use the transmitters to send a wave through a known distance in the sample under test, with the secondary device or devices placed at a known distance from the hand-held unit. Speed in this case equals the distance travelled through the structure divided by the time for the wave to pass from the roving transducer back to the transducer assembly, there being no reflection involved so no correction to the time measured. The experimentally determined speed of sound can then, preferably but not essentially, be used by the inspection device to scale or size the images it creates more accurately.

It is preferable but not necessary to have means on or with the inspection device to place a mark on the surface of the structure under inspection at a time and position chosen by the operator. The mark can be used to identify a point so that the transducer assembly can be removed and replaced or to identify a point where some feature of interest has been found during inspection that requires further inspection or repair or for any other purpose.

It is preferable but not essential to have a handle on the transducer array of the inspection device so that the operator can hold it conveniently. It is also preferable that the handle can be adjusted to suit the needs of the operator whilst she or he is using the transducer assembly in any particular orientation.

It is preferable but not essential that there is one or possibly a plurality of data processing devices in an inspection device, preferably but not necessarily: a computer or a microprocessor or a digital signal processor or a microcontroller or combinations of the aforementioned devices along with suitable memory and other electrical circuits. The said data processing devices preferably but not necessarily control some or all of the following functions: the transmission of any sound and ultrasonic waves, the receiving of any sound or ultrasound waves, the conversion of any received waves into electrical and digital signals, recording of signals, the processing of signals, the creation of images, selection of transducers in any arrays for transmitting and receiving, calculating the position of any transducer array, responding to button presses by the operator and any other processing that may be required by an inspection device.

It is preferable but not essential for an inspection device to have a sealable enclosure to contain some or preferably all of the electronic circuits and any batteries used in the inspection device but not forming essential parts of a transducer assembly. It is preferable that the electronic unit is rugged and capable of being sealed by the operator or a service engineer against water and other environmental effects such as dust. Being a substantially sealed unit it is preferable but not essential that the electronic unit has air circulating freely about as much of it as possible to allow any heat dissipated by electrical circuits therein as large a surface as possible through which to liberate heat.

It is preferable but not essential that the sealable enclosure can be installed easily in a back-pack and worn by the operator.

It is preferable but not essential that all signal processing is done quickly, within a few seconds, so that the operator can respond to the results and either stop testing or repeat tests, possibly at slightly different locations, to improve the quality of image.

In an alternative embodiment, with substantially the same signal processing as described herein to generate images of the interior of the sample under test, a mechanically simpler system is used to position a few transducers, not less than two, at a plurality of test locations. This embodiment is generally slower in use and does not provide the interactive speed and ergonomic advantages of the transducer assemblies already described. Means are provided to set the transducers at specific locations, preferably at random or semi-random positions. It is preferable that there should be some degree of randomness in the position of transducers because the spatial Fourier transform of periodically positioned transducers has periodically spaced peaks that could result in distortions in any images or image-artefacts. In this case the display unit may be separate from the transducer assembly and could form part of a mobile computer, which would also perform some or all of the signal processing. In this embodiment the operator moves the transducers individually or in convenient groups from position to position and to some degree the operator has responsibility for controlling the positions of transducers and in synchronizing positions with the expectations of position for the signal processing. One possible embodiment is a thin sheet of flexible material with holes positioned on a random or semi-random pattern through which transducers can be inserted; another possibility is to project a light pattern onto the structure being inspected and to position the transducers on positions illuminated.

### SPECIFIC DESCRIPTION

Three specific embodiments of the invention will now be described.

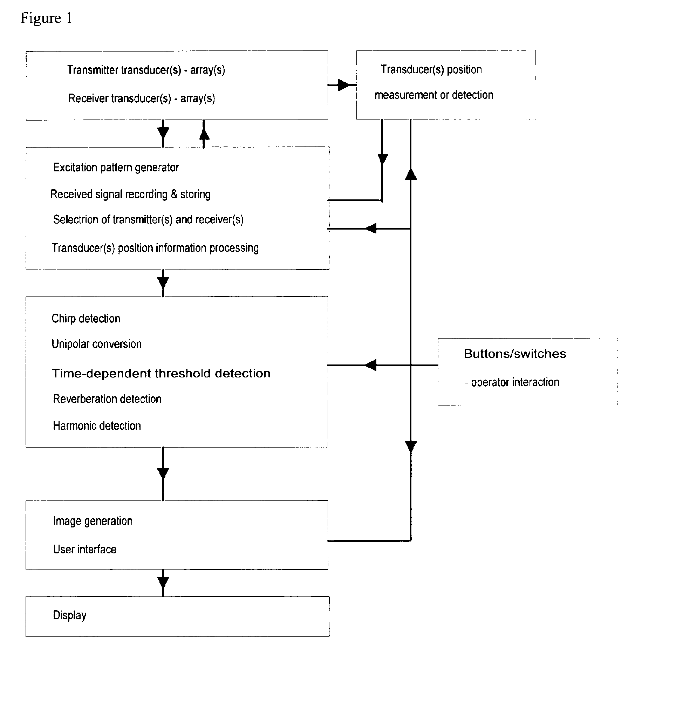

Figure 1 shows a block diagram of the main components and flow of information within an inspection device that is substantially but not essentially common to all embodiments.

The first embodiment will now be described with reference to the accompanying drawings in which:

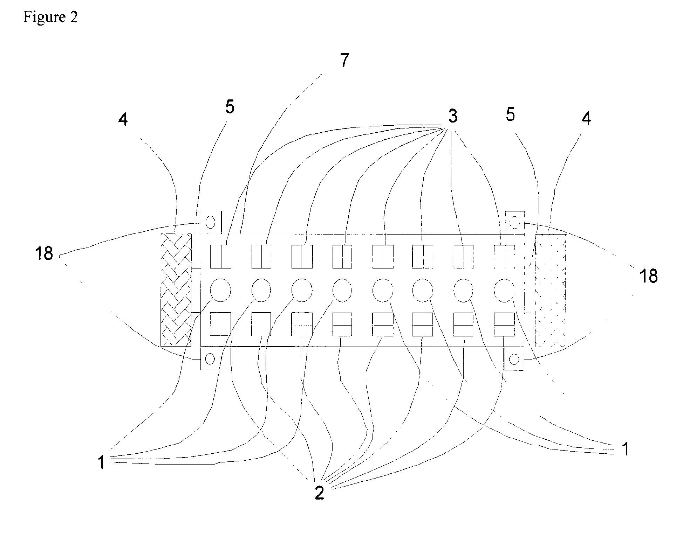

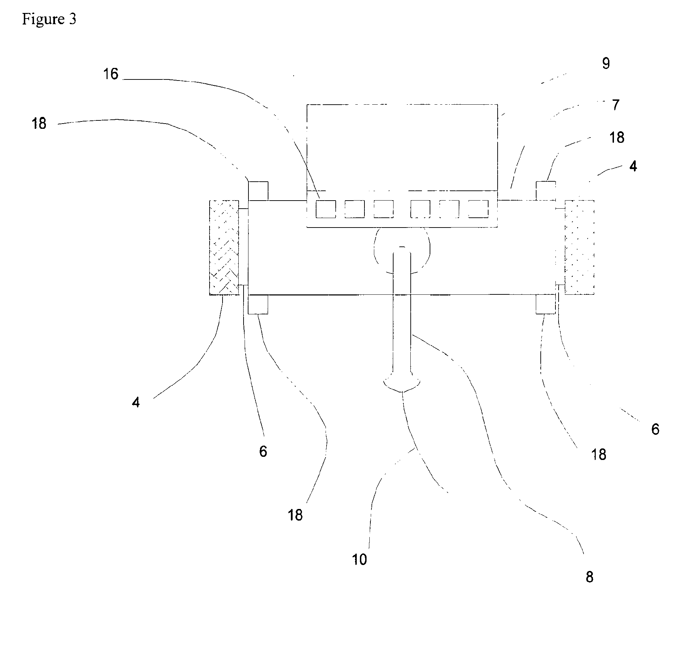

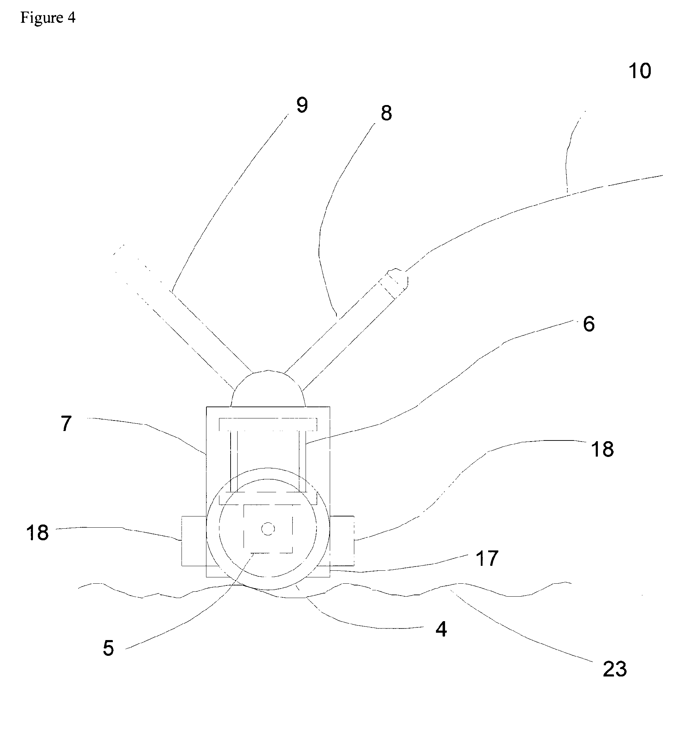

Figures 2, 3 and 4 show three different projection sketches of the principal parts of a lightweight transducer assembly containing three linear arrays of transmitting and receiving transducers forming part of an inspection device, for use carried and held in place by an operator's hand or hands. Figure 2 shows a view of the transmitting and receiving face of the transducer assembly, without a coupling agent, which face engages with the surface of the structure to be inspected. Figure 3 shows the top of the transducer assembly, normally seen by the operator during testing. Figure 4 shows a side view of the transducer assembly, with a coupling layer, 17, attached; normally obscured parts are shown with dashed lines.

Figure 5 shows a sketch of a sealed electronic unit forming part of the inspection device, for use carried on the operator's back and connected by a multi-way cable to the transducer assembly.

Referring to the drawings, the inspection device comprises: three linear arrays of transducers: an array of transducers, 1, substantially sensitive to compression waves only; an array of transducers, 2, substantially sensitive to shear waves parallel to the line of the array only; and an array of transducers, 3, substantially sensitive to shear waves perpendicular to the line of the array only; each array comprising 8 individual transducers; each transducer retained in a housing, 7, but able to move approximately 1 centimetre perpendicular to the surface under test, 23, against a modest reaction force sufficient to cause each transducer to engage the surface of the structure effectively through any coupling layer, 17; with the housing, 7, connected to two sliding assemblies, 6, one at each end of the housing, 7, upon each sliding assembly, 6, is mounted a shaft encoder, 5, upon each shaft of which there is a wheel, 4, with rubber tyres, which are each free to rotate independently as the transducer assembly is pushed or pulled across the surface under test, 23; a display panel, 9, able to display images of the interior of the structure as calculated by the inspection device, with control switches, 16, and the display panel, 9, able to move and rotate to various positions convenient for the operator; a handle, 8, able to rotate to different positions convenient for the operator, from which emerges a cable, 10, that makes electrical connections between the transducer assembly and the electronic circuits held in the back-pack, 12, 13, 15; a back-pack with a sealable enclosure, 15, for electronic circuits; straps, 12, to hold the back-pack onto the operator's back (operator 26); spacers, 13, to keep the electronic unit away from the operator's back to maximize cooling of the electronic circuits, 15; a coupling material, 17, to convey sound waves or ultrasound waves between the transducers, 1, 2, 3, and the surface of the structure, 23; ink-jet droppers, 18, to deposit ink marks onto the surface under test showing the position of the four corners of the transducer housing, 7, during a test.

In use the signal processing unit inside the enclosure, 15, has a charged battery. The operator, 26, puts the back-pack, 12, 13, 15, on his back and adjusts the straps, 12, to hold the back-pack in place. The transducer assembly is connected to the signal processing unit using the cable, 10. The operator, 26, takes the transducer assembly in her or his hand and operates a switch on the inspection device, 16, which causes the inspection device to be energized by the battery and to become functional. A sequence of instructions runs on the processor unit within the electronic circuits, with the operator, 26, controlling some aspects of the processing via the display panel, 9, and the control switches, 16. The operator next places the two wheels, 4, of the transducer assembly in contact with the structure to be inspected on the surface of interest, 23, and moves the transducer assembly by causing the wheels to rotate to a location for inspection. The operator then causes the transducer assembly to engage with the surface of interest, 23, through a coupling layer, 17, by the sliding action of the sliding assembly, 6, and with the operator applying sufficient force through the handle, 8, to enable the sound waves or ultrasound waves to flow through any coupling layer, 17, between the transducers, 1, 2, 3, and the structure; by interacting with the signal processing, the operator causes a suitable selection of one or more of the transducers of type, 1, 2 or 3, to be electrically excited in turn with suitable chirp signals so that they create sound waves or ultrasound waves that are to a substantial degree wave representations of the chirp. The waves travel into the structure under test and echoes from its surface and from the interior structure beneath the surface return to the transducers 1, 2, 3 where one or more of them act as receivers to create electrical signals from the echo-waves. The electrical signals are conveyed to the signal processing unit for processing and storing and conversion into an image. The display unit, 9, indicates to the operator when all sonic or ultrasonic activity in the transducer assembly has finished and it displays any image that the operator selects to display using the control switches, 16. The operator may choose to repeat the test at the same location or disengage the coupling layer, 17, and transducers, 1, 2, 3, from the test surface by using the sliding action of the sliding assembly, 6, in reverse, then, while keeping the wheels, 4, in contact with the test surface, move the transducer assembly to another location of interest, causing the wheels, 4, to rotate and the shaft encoders, 5, connected to them to rotate too. The shaft encoders, 5, create electrical signals which are registered by electronic circuits in the inspection device, from which signals the new position and orientation of the transducer assembly can be calculated. The operator can then repeat the process of collecting information from the new test location and use this information to improve the quality of the image from the first test location.

In use the processor inside the electronic circuits can perform various different signal processing or imaging under the control of the operator using the control buttons, 16. The processing is done quickly, within a few seconds, so that the operator can review the results and respond to them: possibly to stop testing, or possibly by moving to a different location to collect more information and combine the new information with the information used to make the existing image and thereby improve the quality of the image. The operator can also choose to mark the location of the transducer housing, 7, on the test surface by actuating the ink drop markers, 18, for the purpose of identifying a chosen location possibly for additional inspection or repair work.

The second embodiment will now be described with reference to the accompanying drawings in which:

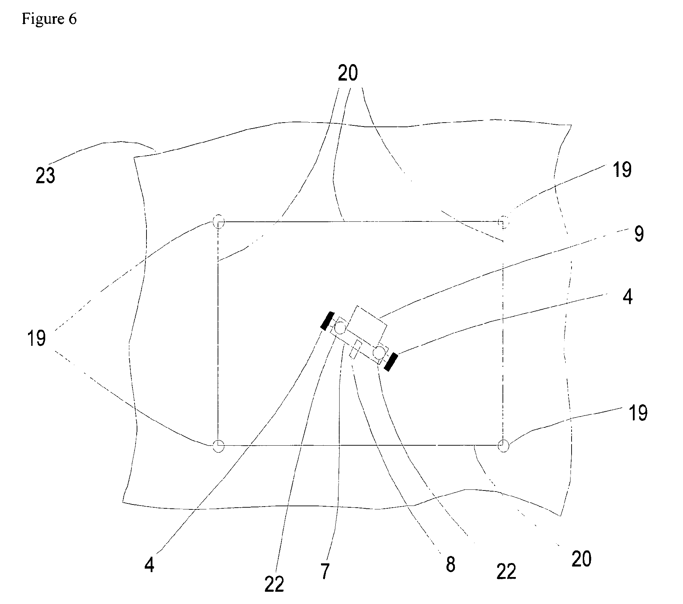

Figure 6 shows a sketch of the transducer assembly, substantially as described in Figures 2, 3 and 4 but shown with reduced detail here for ease of illustration.

The transducer assembly can be identified from its wheels, 4, transducer housing, 7, display unit, 9, controlling handle, 8. It is in use on a structure surface, 23.

Specific additional functionality on the transducer assembly are two sound wave or ultrasound wave transducers, 22, for working in air and not into the structure under test, which are caused by electrical circuits forming part of the inspection device to emit or receive ultrasonic pulses in the air. Four sound or ultrasound transducers working into air, 19, are temporarily attached to the surface under test at known positions and substantially the same average height above the surface as the transducers, 22, on the transducer assembly when engaged with the surface under test. The purpose of the transducers, 19 and 22, is to work together transmitting sound or ultrasound pulses in air between them to determine the position of the transducer assembly and its orientation with reference to the four fixed transducers, 19. Cables, 20, electrically connect transducers, 19, with the other parts of the inspection device and with each other. The advantage of this arrangement is that the transducer assembly can be removed from contact with the surface under test and returned with no loss of registration of the position of the transducer assembly.

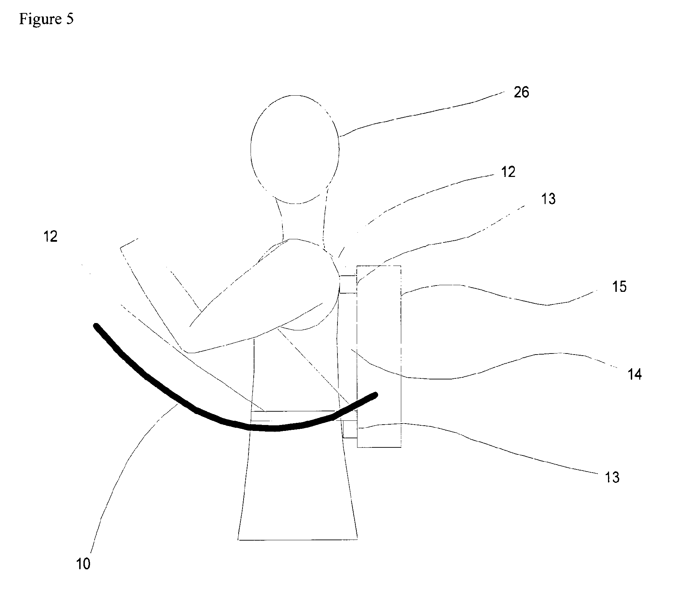

The electronic unit shown in Figure 5 is also used with the components shown in Figure 6.

The third embodiment will now be described with reference to the accompanying drawings in which:

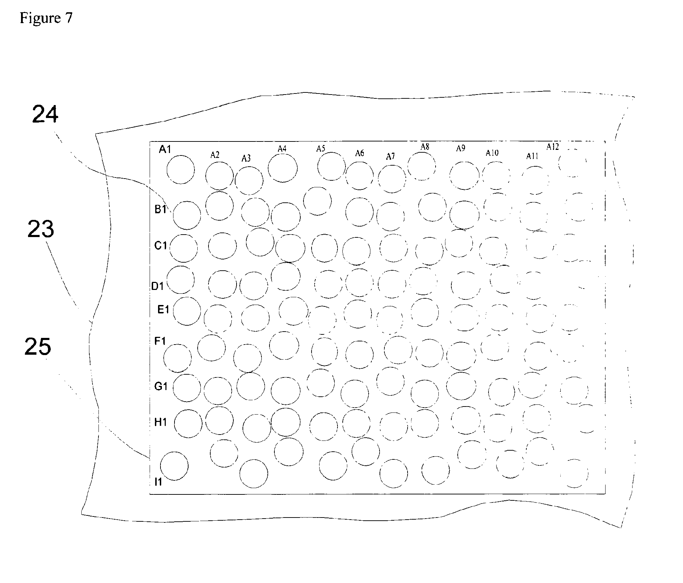

Figure 7 shows a sketch of a flexible, thin, sheet material, 25, used to define an semi-random arrangement of test holes for transducers, each hole uniquely identified by a label, only some of which are shown in figure 7, into which holes the operator inserts transducers and holds them during testing. In use the sheet is first held in place or fixed temporarily to the surface of the structure under test and then individual transducers are inserted into the holes in the sheet by the operator either singly, or two transducers are inserted at different positions simultaneously, or a group of transducers held in a convenient single unit is inserted with a constrained spacing between them, such as a linear array or a square array or some other shape of array. The operator inserts the transducers either in a pattern determined in advance to suit the signal processing or the operator chooses the positions but informs the signal processing algorithm of each position with reference to the unique identifier-labels by some suitable means, such as keypad entries or voice recognition. In other respects the operation of the signal processing to generate an image is substantially as described herein.

The electronic unit shown in Figure 5 is also used with the components shown in Figure 7.

## Claims

1. A device for inspecting the mechanical integrity and operational worthiness of structures, including structures made of heterogeneous materials, using sound waves or ultrasound waves and their echoes from components within the structure, with: (a) means for geometrically arranging one or more but few sonic or ultrasonic electro-mechanical conversion transducers, (b) means to select any of the transducers and means to cause any one or a few at a time of the selected transducers to generate sound waves and/or ultrasound waves and means to select any of the transducers and means to collect electrical signals received from any one or a few at a time of the selected transducers caused by the impingement of sound waves and/or ultrasound waves echoing from the structure under inspection; (c) the aforementioned transducers with some or possibly all able to generate and/or receive substantially compression sound waves or ultrasound waves, or with possibly some or possibly all of the aforementioned transducers able to generate and/or receive substantially shear sound waves or ultrasound waves; (d) with the direction or plane of shear polarization of the aforementioned shear-transducers all being known and constrained either parallel or of a known pattern of polarization between each individual transducer; (e) each aforementioned transducer with an aperture that is preferably not larger and possibly much smaller in actuating dimension than a representative wavelength of sound wave and/or ultrasound wave used for inspection; (f) all the aforementioned transducers with average actuating surface level, when in use, being possibly co-planar or possibly profiled to match the profile of the surface of the structure to be inspected, and possibly with means to adapt the profile to the profile of the surface of the structure; (g) the aforementioned transducers provided with means to register the position of each transducer on the surface of the structure when a test is performed so that this information can be used along with the received echoes from the structure to form images of the interior of the structure; (h) the aforementioned transducers provided with means to allow them to move or slide substantially perpendicular to the surface under test and to be engaged with and to disengage from the testing surface of the structure under inspection; (i) means to couple or transfer sound wave or ultrasound wave energy between each aforementioned transducer and the surface of the structure under inspection, when engaged thereupon; (j) means for creating and applying excitation patterns to a transducer selected to transmit; (k) means for collecting and storing representations of signals received by selected transducers; (1) means for processing the signals from receivers to create an image to represent the interior of the structure under test based upon echoes from sound waves or ultrasound waves emitted into the structure by the inspection device; (m) means to display information about the interior of the structure under test for an operator to view; (n) means to allow an operator to control the conditions of testing; (o) means to provide power to the inspection device; (p) means to allow the inspection device to be used quickly and easily to inspect a structure.

2. A device for inspecting the mechanical integrity and operational worthiness of a structure from a single surface substantially as claimed in Claim 1 that is portable, lightweight and capable of performing tests quickly and continuing to work for sufficient time to allow at least one inspection to be performed on a structure and preferably but not essentially continuing to work for several hours.

3. A device for inspecting the mechanical integrity and operational worthiness of a structure from a single surface substantially as claimed in Claims 1 and 2 wherein there is a group or groups of transducers mechanically held together in one or more housings, to make transducer assemblies, such that the group consists of at least two or more transducers in a line, with a space between each of approximately one transducer width or commensurate with the wavelength of sound wave or ultrasound wave used in testing.

4. A device for inspecting the mechanical integrity and operational worthiness of a structure from a single surface substantially as claimed in Claims 1 to 3 wherein there is at least one or more and preferably three groups of transducers mechanically held together in a housing, thereby creating a transducer assembly, such that each group of transducers consists of at least two or more in a line, with a space between each transducer, and the lines parallel and kept close together; one group of transducers would be preferably but not essentially, substantially sensitive to compression waves, a second group would be preferably but not essentially substantially sensitive to shear waves each with their plane of polarization parallel to the line of the transducers, a third group would be preferably but not essentially be substantially sensitive to shear waves each with their plane of polarization perpendicular to the line of the transducers; in this preferred embodiment the inspecting device can be operated in up to nine different inspection modes: transmitting compression waves and receiving shear parallel waves, transmitting compression waves and receiving shear perpendicular waves, transmitting compression waves and receiving compression waves, transmitting shear parallel waves and receiving compression waves, transmitting shear parallel waves and receiving shear perpendicular waves, transmitting shear parallel waves and receiving shear parallel waves, transmitting shear perpendicular waves and receiving compression waves, transmitting shear perpendicular waves and receiving shear parallel waves and transmitting shear perpendicular waves and receiving shear perpendicular waves, with each mode being used at the operator's wish to create an image of the interior of the structure under test.

5. A device for inspecting the mechanical integrity and operational worthiness of a structure from a single surface substantially as claimed in Claims 1 to 4 wherein there is a mechanical lifting device or arm used for lifting and moving possibly but not essentially heavier embodiments of the inspection device or parts thereof, or an arm possibly but not essentially in the form of a pantograph that can be used to register the position and orientation of the transducer assembly relative to the surface under test.

6. A device for inspecting the mechanical integrity and operational worthiness of a structure from a single surface substantially as claimed in Claims 1 to 5 wherein the transducer assembly is portable, lightweight and capable of being held by an operator.

7. A device for inspecting the mechanical integrity and operational worthiness of a structure from a single surface substantially as claimed in Claims 1 to 6 wherein some or possibly all of any electrical circuits and any batteries are held in an enclosure separate from the any transducer assembly.

8. A device for inspecting the mechanical integrity and operational worthiness of a structure from a single surface substantially as claimed in Claims 1 to 7 with a hand-held transducer assembly having relatively few transducers that are mostly or all relatively lightweight.

9. A device for inspecting the mechanical integrity and operational worthiness of a structure from a single surface substantially as claimed in Claims 1 to 8 with a hand-held transducer assembly having an array of approximately four to ten transducers in a line (a linear array), which by rotating its orientation can be used on structures containing internal or surface components or flaws or features of linear shape to discover the orientation of the interior linear features from the surface.

10. A device for inspecting the mechanical integrity and operational worthiness of a structure from a single surface substantially as claimed in Claims 1 to 9 with a plurality of transducers in one or more linear arrays but each transducer with the capability to move substantially perpendicular to the line of the array and substantially perpendicular the surface under test and for each transducer to move substantially independently, which motion allows the linear array of transducers to be used on different test surfaces, including: flat surfaces, cylindrical surfaces, many other surface profiles and rough surfaces.

11. A device for inspecting the mechanical integrity and operational worthiness of a structure from a single surface substantially as claimed in Claims 1 to 10 with means to combine signals received from transducers at two or more positions to create an image of the interior of the structure under test and in so doing to reduce substantially the otherwise obfuscating effect of scattering of sound waves or ultrasound waves, particularly the random scattering found in heterogeneous materials.

12. A device for inspecting the mechanical integrity and operational worthiness of a structure from a single surface substantially as claimed in Claims 1 to 11 with means for creating an image which is at a focal plane located in the interior of the structure under test, chosen by an operator, with the focal plane divided into a plurality of focal points, possibly again chosen by the operator; for each focal point as many stored echo signals as possible, or as many as thought desirable by the operator, are processed and appropriate parts of them added together, the appropriate parts chosen with regard to compensating time-delays chosen to compensate for the geometrical distances travelled by sound waves or ultrasound waves in the structure to create a focussed intensity at each focal point and by repeating the process for every focal point in the focal plane a focussed image is developed over the entire focal plane which is substantially sharp; by setting a focal plane inside the structure and at different depths or orientations, different images of the interior of the structure can be viewed by the operator.

13. A device for inspecting the mechanical integrity and operational worthiness of a structure from a single surface substantially as claimed in Claims 1 to 12 with a linear array of transducers in which the total extent of the distance covered by the transducers along the line is, preferably but not essentially, several times longer than the grain size of the material under inspection and, possibly but not necessarily, several times longer than the wavelength of the shortest wavelength of sound wave or ultrasound wave transmitted in an inspection by the inspection device, with a spacing between transducer centres of either the aperture distance or roughly one or at most a few wavelengths representative of the sound waves or ultrasound waves generally used for inspection by the inspection device.

14. A device for inspecting the mechanical integrity and operational worthiness of a structure, possibly but not necessarily a concrete structure, from a single surface substantially as claimed in Claims 1 to 13 with a linear array of transducers for use on concrete, of length not substantially less than 100 millimetre and not substantially more than 500 millimetre long.

15. A device for inspecting the mechanical integrity and operational worthiness of a structure from a single surface substantially as claimed in Claims 1 to 14 with an assembly of two or more transducers that can be moved over the surface of the structure under test so that a plurality of testing locations using sound waves or ultrasound waves can be accessed, with means to measure the test locations of the transducers.

16. A device for inspecting the mechanical integrity and operational worthiness of a structure from a single surface substantially as claimed in Claims 1 to 15 with an assembly of two or more transducers that can be moved over the surface of the structure under test using one or more wheels or rollers or track mechanisms or similar moving means at opposite ends of the assembly of transducers, with the wheels or rollers or track mechanisms or similar moving means in contact with the surface of the structure under test and with means for the revolving of the wheels or rollers or track mechanisms or similar moving means to be measured, so that it is possible to determine the relative position of each and every transducer in the assembly at the various test locations and means for the transducers to engage with the surface under test, possibly but not necessarily, using a sliding mechanism acting in a direction substantially perpendicular to the test surface.

17. A device for inspecting the mechanical integrity and operational worthiness of a structure from a single surface substantially as claimed in Claims 1 to 16 with an assembly of two or more transducers that can be moved over the surface of the structure under test, with, attached to the assembly, at least one but preferably two or more sonic or ultrasonic transducers working in air and with two or more and preferably four sonic or ultrasonic transducers working in air substantially fixed temporarily at two or more or preferably four known positions on the surface of the structure under test and suitably separated by known distances to allow the transducer assembly to be used substantially within the extent of the fixed transducers at as many positions as desired by the operator for one region of interest on the structure under test, all with means for the transducers to pass sound waves or ultrasound between them in air and to pass electrical signals by cable and to electronic circuits for signal processing to determine the position of the transducer assembly using the time taken for the sound waves or ultrasound waves to travel in air between the several aforementioned transducers with an accuracy of measuring the position of the mobile transducer assembly substantially smaller than the smallest wavelength of sound wave or ultrasound wave transmitted into the sample under test.

18. A device for inspecting the mechanical integrity and operational worthiness of a structure from a single surface substantially as claimed in Claims 1 to 17 with an assembly of two or more transducers that can be moved over the surface of the structure under test, with attached to the assembly at least one but preferably two or more radio-wave transceivers and with, preferably but not essentially, two or more and preferably four radio-wave transceivers substantially fixed temporarily at two or more or preferably four known positions on the surface of the structure under test and suitably separated by distances to allow the transducer assembly to be used substantially within the extent of any fixed transceivers at as many positions as desired by the operator for one region of interest on the structure under test, all with means for the radio-wave transceivers to pass signals in air by electro-magnetic waves and by electrical signals through cables to electronic circuits and signal processing to determine the position of the transducer assembly using the time taken for the radio waves to travel between the several aforementioned radio transceivers with an accuracy of measuring the position of the mobile transducer assembly substantially smaller than the smallest wavelength of sound wave or ultrasound wave transmitted into the structure under inspection.

19. A device for inspecting the mechanical integrity and operational worthiness of a structure from a single surface substantially as claimed in Claims 1 to 18 with means for coupling or transferring ultrasonic energy between each transducer and the surface under test, using point contact.

20. A device for inspecting the mechanical integrity and operational worthiness of a structure from a single surface substantially as claimed in Claims 1 to 19 with means for coupling or transferring ultrasonic energy between each transducer and the surface under test, using solid or substantially solid but pliable materials including: adhesives, fast-setting mortar and deformable solid materials.

21. A device for inspecting the mechanical integrity and operational worthiness of a structure from a single surface substantially as claimed in Claims 1 to 20 with transducers with an aperture size that is not substantially greater than one wavelength of the highest frequency of sound or ultrasound used in an inspection of a structure.

22. A device for inspecting the mechanical integrity and operational worthiness of a concrete structure from a single surface substantially as claimed in Claims 1 to 21 with transducers with an aperture size that is not substantially greater than 50 millimetre and preferably not substantially greater than 20 millimetre.

23. A device for inspecting the mechanical integrity and operational worthiness of a structure from a single surface substantially as claimed in Claims 1 to 22 with transducers with acoustic impedance of the material forming the transmitting surface matched to the acoustic impedance of the structure under test and made of a material that is compatible with any coupling material.

24. A device for inspecting the mechanical integrity and operational worthiness of a structure from a single surface substantially as claimed in Claims 1 to 23 with transducers with a small dome or protrusion or a plurality of possible protruding shapes of characteristic size commensurate or slightly greater than the surface roughness of the surface under test, which provides means for point-contact coupling while remaining compatible for use with liquid coupling materials, adhesives and substantially solid coupling materials, so that the operator has a choice of coupling material to use to suit the prevailing test requirements and conditions of the surface to be tested.

25. A device for inspecting the mechanical integrity and operational worthiness of a structure from a single surface substantially as claimed in Claims 1 to 24 with means to avoid using transducers simultaneously as receivers and transmitters thereby retaining maximum information from the interior of the structure under test.

26. A device for inspecting the mechanical integrity and operational worthiness of a structure from a single surface substantially as claimed in Claims 1 to 25 with means to process the resulting signals and display the results to the operator preferably but not essentially within a few seconds of performing a test, in order to reduce the time that the operator or any other mechanical means holds a transducer array in contact with the test surface and so that the operator or inspection engineer is able to assess the information resulting from any given test quickly, without leaving the site to create images away from the structure, and to decide if testing at another location is desirable, thereby enabling an interactive and more efficacious procedure of inspection.

27. A device for inspecting the mechanical integrity and operational worthiness of a structure from a single surface substantially as claimed in Claims 1 to 26 with preferably but not essentially relatively few electronic channels with which to amplify signals to and from transducers so that preferably but not essentially only one transducer is a transmitter and preferably but not essentially only one transducer is a receiver at a time in order to reduce the replication of electronic circuits thereby to reduce the cost and weight of the device.

28. A device for inspecting the mechanical integrity and operational worthiness of a structure from a single surface substantially as claimed in Claims 1 to 27 with means to drive transmitter transducers with electrical excitation in a controlled pattern, which pattern is chosen to suit the requirements of the inspection, the material under test and capabilities of the transducers, in particular the useful frequency bandwidth but otherwise, preferably but not essentially, having as wide a range of frequencies as possible.

29. A device for inspecting the mechanical integrity and operational worthiness of a structure from a single surface substantially as claimed in Claims 1 to 28 with means to drive transmitter transducers with electrical excitation substantially in the form of swept-frequency chirps, comprising bursts of sine waves lasting at least the time of one sine cycle during which time the frequency of the sine wave is changed or swept.

30. A device for inspecting the mechanical integrity and operational worthiness of a concrete structure from a single surface substantially as claimed in Claims 1 to 29 with means to drive transmitter transducers with swept-frequency chirps of duration in the range 30 to 300 microseconds, within which time and the frequency sweeps between two frequencies somewhere in the range 200 kHz to 10 kHz.

31. A device for inspecting the mechanical integrity and operational worthiness of a structure from a single surface substantially as claimed in Claims 1 to 30 with means to process echo-signals from receiver transducers to detect occurrences of any pattern sent out by transmitter transducers and to provide an event marker with more precise timing than the duration of the original pattern.

32. A device for inspecting the mechanical integrity and operational worthiness of a structure from a single surface substantially as claimed in Claims 1 to 31 with means to process echo-signals from receiver transducers to detect occurrences of patterns sent from transmitter transducers, in which the processing means is a signal processing algorithm sometimes called matched filtering, a form of cross-correlation, and which uses as the basis of the pattern recognition either a copy of the electrical excitation signal pattern or a recording of a pattern transmitted in a calibrating test on a suitable material using substantially the same transducers and the same excitation chirp as are used in the inspection device on the structure under inspection, so that any modifications to or distortions of the pattern, either caused by the transmitter or by the receiver, can be included in the pattern to be recognized and thereby contribute to a sharper image of the interior of the structure.

33. A device for inspecting the mechanical integrity and operational worthiness of a structure from a single surface substantially as claimed in Claims 1 to 32 with matched filtering or other pattern recognition means to process echo-signals from receiver transducers to detect occurrences of any kind of regular excitation used to drive transmitter transducers, including sharp spike excitation, which results in echo-signals with characteristic patterns that can be recorded in a calibration experiment and using that part of the recording containing the pattern as the basis of the matched filter or other pattern recognition means.

34. A device for inspecting the mechanical integrity and operational worthiness of a structure from a single surface substantially as claimed in Claims 1 to 33 with means to process signals, preferably but not essentially after pattern recognition, and convert them from a bipolar form into a unipolar form, to eliminate phase-cancelling or subtraction in later processing, preferably but not essentially, using the magnitude of the analytic function of the signal or simple rectification or taking the magnitude of the signal to perform unipolar conversion, the latter two methods preferably but not essentially followed by low-pass or band-pass filtering to smooth the signal.

35. A device for inspecting the mechanical integrity and operational worthiness of a structure from a single surface substantially as claimed in Claims 1 to 34 with means to process signals, preferably but not essentially after unipolar conversion, to detect peaks in the signal using a time-varying threshold based upon the statistics of one or more signals received from substantially similar random scattering materials as those that may be in the structure under inspection and, preferably but not essentially, by adjusting the parameters of the statistics to provide a degree of control over the sensitivity of peak detection.

36. A device for inspecting the mechanical integrity and operational worthiness of a structure from a single surface substantially as claimed in Claims 1 to 35 with means to process signals, preferably but not essentially after unipolar conversion, to detect peaks in the signal using a time-varying threshold, preferably but not necessarily, based upon the Weibull or Rayleigh mathematical distributions and by adjusting the parameters of the chosen distribution to provide a degree of control over the sensitivity of peak detection.

37. A device for inspecting the mechanical integrity and operational worthiness of a structure from a single surface substantially as claimed in Claims 1 to 36 with means to process signals derived from receiver signals to identify any reverberations or resonances therein and with means to measure the frequency of those resonances and means to locate where in a structure under test is the probable source of resonance and means to display the position and other characteristics of the resonance on a display to the operator.

38. A device for inspecting the mechanical integrity and operational worthiness of a structure from a single surface substantially as claimed in Claims 1 to 37 with means to process signals to identify therein any harmonics or sub-harmonics of the excitation pattern used to drive transmitter transducers and means to locate where in the structure under test is the probable source of harmonics and means to display the position and other characteristics of the harmonics on a display to the operator.

39. A device for inspecting the mechanical integrity and operational worthiness of a structure from a single surface substantially as claimed in Claims 1 to 38 with a power cable to a source of electrical energy or with its own a source of electrical energy, possibly but not necessarily a re-chargeable battery or batteries, being substantially lightweight so that it or they can be carried by an operator with sufficient energy in one charge to perform at least one inspection and preferably but not essentially sufficient energy in one charge for an inspection device to be used for some hours with the operator substantially free to move about the structure during an inspection, all with relatively little time spent in gathering-up parts of the inspection device.

40. A device for inspecting the mechanical integrity and operational worthiness of a structure from a single surface substantially as claimed in Claims 1 to 39 with means for the operator comfortably to view an image created from a test or tests on a display panel mounted, preferably but not essentially, on the inspection device or possibly held by means of a hat or a band onto the operator's head, preferably but not necessarily while the operator is still holding the transducer assembly in contact with the surface of the structure under test.

41. A device for inspecting the mechanical integrity and operational worthiness of a structure from a single surface substantially as claimed in Claims 1 to 40 with a lightweight display panel mounted on the transducer assembly.

42. A device for inspecting the mechanical integrity and operational worthiness of a structure from a single surface substantially as claimed in Claims 1 to 41 with a lightweight display panel mounted on the transducer assembly that can be adjusted in angle to suit the operator while the inspection device is in use.

43. A device for inspecting the mechanical integrity and operational worthiness of a structure from a single surface substantially as claimed in Claims 1 to 42 with means to measure the speed of sound or ultrasound of compression and/or shear waves in the structure under test using the inspection device, with means for the operator to enter into the inspection device a dimension value on the structure penetrated by the sound wave or ultrasound wave in question or by connecting a secondary or roving transmitter of sound or ultrasound waves to the inspection device and using it to send a wave through a known distance in the sample under test to the inspection device, with the secondary device or devices placed at a known distance from the hand-held unit so that the inspection device can make timing measurements of the wave crossing the known dimension and thereby calculate the speed of sound and subsequently to use the result to scale or size images of the interior of the structure under test.

44. A device for inspecting the mechanical integrity and operational worthiness of a structure from a single surface substantially as claimed in Claims 1 to 43 with means to place a mark or marks on the surface of the structure under inspection at a time and position chosen by the operator.

45. A device for inspecting the mechanical integrity and operational worthiness of a structure from a single surface substantially as claimed in Claims 1 to 44 with means to engage and disengage some or all of the transducers in the transducer assembly from the surface of the structure under inspection, while still maintaining registration of its position and means to move the transducer assembly as one unit over the surface to a new position, preferably but not necessarily nearby, and means to cause the transducer array to re-engage with the surface so that more tests can be done and means to measure the relative distance and orientation of the transducers at the two positions of being engaged.

46. A device for inspecting the mechanical integrity and operational worthiness of a structure from a single surface substantially as claimed in Claims 1 to 45 with means to hold the transducer assembly, such as a handle.

47. A device for inspecting the mechanical integrity and operational worthiness of a structure from a single surface substantially as claimed in Claims 1 to 46 with means to hold the transducer assembly, such as a handle, and further means to adjust the position of the holding means or handle relative to the transducer assembly to suit the needs of the operator or the test.

48. A device for inspecting the mechanical integrity and operational worthiness of a structure from a single surface substantially as claimed in Claims 1 to 47 with one or possibly a plurality of data processing devices in an inspection device, preferably but not necessarily: a computer or a microprocessor or a digital signal processor or a microcontroller or combinations of the aforementioned devices along with suitable memory and other electrical circuits and means needed for an inspection device; the said data processing devices preferably but not necessarily with means to control some or all of the following functions: the transmission of any sound and ultrasonic waves, the receiving of any sound or ultrasound waves, the conversion of any received waves into electrical signals and preferably but not necessarily into digital signals, recording of signals, the processing of signals, the creation of images, selection of transducers in any arrays for transmitting and receiving, calculating the position of any transducer array, responding to button presses by the operator and any other processing needed by an inspection device.

49. A device for inspecting the mechanical integrity and operational worthiness of a structure from a single surface substantially as claimed in Claims 1 to 48 with means for enclosing, preferably but not necessarily in a sealed enclosure, the electronic circuits and related components not included in the transducer assembly so that the circuits and components therein can operate correctly under differing weather and environmental conditions including rain and dust.

50. A device for inspecting the mechanical integrity and operational worthiness of a structure from a single surface substantially as claimed in Claims 1 to 49 with means for enclosing the electronic circuits and related components not included in the transducer assembly, in an enclosure held in a back-pack that can be carried conveniently by the operator in all weather and environmental conditions and, preferably but not essentially, with means to hold the enclosure away a short distance from the operator's back to permit air to circulate all around the enclosure and thereby help to keep down both the temperature of the enclosure and the operator.

51. A device for inspecting the mechanical integrity and operational worthiness of a structure from a single surface substantially as claimed in Claims 1 to 50 with means to position a few transducers, not less than two at various test locations, and with means to set the transducers at specific locations, preferably but not necessarily at random or semi-random positions having spacing greater than the aperture of the transducers, with the operator or some mechanical means moving the transducers to different positions and with means to provide information about the precise locations of transducers during tests.

52. A device for inspecting the mechanical integrity and operational worthiness of a structure from a single surface substantially as claimed in Claims 1 to 51 with a sheet of material with holes at random or semi-random positions, into which a few transducers, not less than two at a time can be inserted therein by an operator or by mechanical means.

53. A device for inspecting the mechanical integrity and operational worthiness of a structure from a single surface substantially as claimed in Claims 1 to 52 with a display unit, possibly separate from the transducer assembly, forming a component in a mobile computer, which would also perform some, or all, of the signal processing used to create an image.

54. A device for inspecting the mechanical integrity and operational worthiness of a structure from a single surface substantially as claimed in Claims 1 to 53 and substantially as described hereinbefore, especially with reference to one or more of the Figures shown in the accompanying drawings.
